(19) Europäisches Patentamt
European Patent Office
Office européen des brevets

(11) EP 1 455 832 B1

(12) **EUROPEAN PATENT SPECIFICATION**

(45) Date of publication and mention of the grant of the patent:
**20.02.2008 Bulletin 2008/08**

(21) Application number: **02790247.7**

(22) Date of filing: **20.12.2002**

(51) Int Cl.:
***A61K 47/36*** *(2006.01)* ***A61K 9/36*** *(2006.01)*
***A61K 9/62*** *(2006.01)*

(86) International application number:
**PCT/BE2002/000195**

(87) International publication number:
**WO 2003/053470 (03.07.2003 Gazette 2003/27)**

(54) **PULSED BIO-AGENT DELIVERY SYSTEMS BASED ON IN VIVO DEGRADABLE AND SWELLABLE MODIFIED DEXTRAN HYDROGELS**

AUF IN VIVO ABBAUBAREN UND QUELLBAREN MODIFIZIERTEN DEXTRANHYDROGELEN BASIERENDE SYSTEME ZUR STOSSWEISEN FREISETZUNG BIOLOGISCH AKTIVER STOFFE

SYSTEMES DE DISTRIBUTION D'AGENTS BIOLOGIQUES PAR IMPULSIONS BASES SUR DES HYDROGELS DE DEXTRANE MODIFIE EXPANSIBLES ET DEGRADABLES IN VIVO

(84) Designated Contracting States:
**AT BE BG CH CY CZ DE DK EE ES FI FR GB GR IE IT LI LU MC NL PT SE SI SK TR**

(30) Priority: **21.12.2001 GB 0130518**

(43) Date of publication of application:
**15.09.2004 Bulletin 2004/38**

(73) Proprietor: **Universiteit Gent**
**9000 Gent (BE)**

(72) Inventors:
- **DEMEESTER, Jo**
  **B-9040 Sint-Amandsberg (BE)**
- **DE SMEDT, Stefaan**
  **B-9030 Mariakerke (BE)**
- **STUBBE, Barbara**
  **B-9860 Balegem-Oosterzele (BE)**

(74) Representative: **Bird, Ariane et al**
**Bird Goën & Co**
**Klein Dalenstraat 42A**
**3020 Winksele (BE)**

(56) References cited:
**WO-A-01/34677** **WO-A-97/04747**
**US-A- 4 871 549**

- **FRANSSEN O ET AL: "Degradable dextran hydrogels: controlled release of a model protein from cylinders and microspheres" JOURNAL OF CONTROLLED RELEASE, ELSEVIER SCIENCE PUBLISHERS B.V. AMSTERDAM, NL, vol. 60, no. 2-3, 5 August 1999 (1999-08-05), pages 211-221, XP004362935 ISSN: 0168-3659**
- **A.L. GENNARO: "Remington: The Science and Practice of Pharmacy" 15 December 2000 (2000-12-15) , LIPPINCOTT, WILLIAMS & WILKINS , PHILADELPHIA, US XP002245787 * 20th edition, Chapter 47 page 912, right-hand column, last paragraph -page 913, left-hand column, paragraph 3**
- **DE SMEDT STEFAAN C ET AL: "Swelling pressure observations on degrading dex-HEMA hydrogels" MACROMOLECULES; MACROMOLECULES MAR 26 2002, vol. 35, no. 7, 28 February 2002 (2002-02-28), pages 2501-2505, XP002245786**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to the time-controlled delivery of bio-agents such as therapeutic drugs, proteins, vitamins, hormones, biocides, pesticides and the like. More precisely, the invention relates to the use of *in vivo* degradable polymer solutions or hydrogels for time-controlled or pulsed bio-agent release or delivery systems. In particular, the invention relates to such systems comprising a semi-permeable membrane and a bio-agent containing core, the composition and structure of which allows for single or multiple pulse delivery of the bio-agent.

BACKGROUND OF THE INVENTION

Currently, there is a major interest in pulsed drug delivery in which the pharmaceutical device releases the drug at a pre-programmed time. Pulsed drug release can be achieved in different ways, namely by creating a rigid, semi-permeable membrane around a core comprising the drug and a swellable component. The role of such a membrane is (i) to allow for the transport of small molecules (e.g. water molecules, ions) between the swellable component and the surrounding solution, and (ii) to prevent larger molecules (e.g. proteins, polymeric degradation products) to leave the device. During time, the swelling pressure $\Pi_{SW}$ of the core gradually increases. When $\Pi_{SW}$ exceeds the tensile strength of the membrane, the core ruptures, followed by a sudden release of the drug. This is illustrated for instance in U.S. Patent No. 4,871,549 disclosing a so-called time-controlled explosion system in which drug release is caused by explosion of a membrane after a definite time period (defined as a "lag time"), said system being in the form of beads or granules comprising a core (e.g. made of sucrose) covered with an outer layer of drug (optionally comprising a swelling agent), a further outer layer of disintegrating agent or polyvinyl acetate or polyacrylic acid, and an outer membrane of a water-insoluble coating material. By mixing such systems having different lag times, various release patterns may be achieved such as repeat pattern, zero-order pattern (i.e. uniform rate release), reverse first-order pattern (i.e. release rate increases with time) or a sigmoid pattern.

Other systems are also known in the art. For instance, U.S. Patent No. 3,247,066 discloses a core comprising a mixture of drug and a water-swellable colloid coated with a water-permeable polymer. When used for oral administration, body fluid water permeates the coating, causing the colloid to hydrate and swell and break the outer coating thus releasing the drug. This device however suffers from the inherent defect that the swelling of colloids is greatly influenced by pH. U.S. Patent No. 3,952,741 also discloses an osmotic dispenser wherein a water-permeable membrane surrounds an active agent optionally mixed with an osmotic attractant. U.S. Patent No. 4,933,185 discloses a controlled release system comprising microcapsules having an inner polysaccharide core and an outer ionically interacting skin, a biologically active substance, and an enzyme specifically degrading said core polyssacharide, not the ionically interacting skin, until the outer skin loses its integrity and the microcapsules completely break down. U.S. Patent No. 5,593,697 discloses an implant for parenteral administration comprising (i) a drug preferably contained in a core, (ii) an excipient system comprising one water-soluble, preferably biodegradable, material (e.g. lactose) and one water-insoluble, preferably swellable or disintegrating, material (e.g. sodium starch glycolate or stearic or palmitic acid), and (iii) a polymer film coating adapted (e.g. by the incorporation of a permeability modifying agent such as hydroxypropylmethyl cellulose) to rupture after a lag time, the said outer film being impermeable to peptides, proteins, antigens and the like. In the latter embodiment, the lag time is controlled by varying the thickness of the outer film or by the amount of hydroxypropylmethyl cellulose in the coating film. Genarro A.L. (in " Remington: The Science and Practice of Pharmacy ", 20th edition, Chapter 47, pages 912-913) describes tablets consisting of a core of an osmotically active drug, or a core of an osmotically inactive drug combined with an osmotically active salt surrounded by a semi-permeable membrane containing a small orifice.

In the various drug delivery systems described above, the swelling agents used are non-degradable. There is a need in the art for drug delivery devices with more predictable release profiles. There is also a need in the art for drug delivery devices taking advantage of the biodegradability, hence bio-compatibility, of some of their components. The purpose of the present invention is to address these problems.

Hydrogels are well suited for biomedical applications because of their bio-compatibility, however degradable hydrogels have not yet been proposed as swelling controlled drug delivery components.

Furthermore, U.S. Patent No. 5,654,006 discloses a composition for parenteral administration, including encapsulated microparticles having an average size between 0.05 and 5 μm, for rapid release of a therapeutic compound when the composition is exposed to a selected target condition related to pH, temperature or the presence of a selected ligand. The microparticle and entrapped drug are encapsulated within a lipid bilayer membrane. Localized disruption of the lipid membrane, and influx of monovalent ions into the polymer matrix, in response to the selected target conditions, causes a cascade effect involving matrix swelling and further membrane disruption. This composition however suffers from the limitation that a change in ionic environment is required for membrane disruption.

One problem addressed by the present invention is to provide a drug delivery system based on membrane disruption wherein the latter occurs independently from any change in the biological environment.

## SUMMARY OF THE INVENTION

The present invention is based on the principle that in a bio-agent delivery or release system comprising a semi-permeable membrane surrounding a core comprising the drug and a swellable component, the lag time may be suitably controlled by the design and proper selection of an *in vivo* degradable swelling agent rather than by the complicated procedures of tailoring some features, such as composition and thickness, of the membrane, or by only tailoring such features. For the proper development of such delivery devices with predictable release profiles a detailed understanding of the thermodynamic and kinetic properties of degrading hydrogel systems is required.

Based on this underlying principle, the present invention provides the use of an *in vivo* degradable and swellable dextran hydrogel, wherein said dextran hydrogel is a dextran modified by means of at least one ($C_{1-8}$alkyl) acrylate or methacrylate, or at least one (hydroxy-$C_{1-8}$alkyl) acrylate or methacrylate, and wherein degradation occurs by cleavage (i.e. usually hydrolysis) of the oligomer or polymer backbone and/or, in the case of a hydrogel, by cleavage (i.e. usually hydrolysis) of cross-linking bonds within the said hydrogel, as a swellable component of a time-controlled explosion bio-agent release system or a pulsed bio-agent (biologically active agent) delivery system comprising at least one biologically active agent or bio-agent and an outer lipid or polymer membrane, which is permeable to ions and water but impermeable to the bio-agent, wherein the bio-agent release or delivery begins after a lag time ranging from 1 hour to 2 weeks.

## BRIEF DESCRIPTION OF THE DRAWINGS

Figure 1 shows the variation of the amount of free dextran as a function of time in degrading hydrogels of dextran modified by means of hydroxyethyl methacrylate (hereinafter referred to as dex-HEMA) with various degrees of substitution (i.e. number of HEMA groups per 100 glucopyranose residues of dextran, hereinafter referred to as DS) and various concentrations.

Figure 2 shows the variation of the elastic modulus G' as a function of time in degrading dex-HEMA hydrogels with various DS and concentrations.

Figure 3 shows the swelling pressure $\Pi_{sw}$ as a function of the polymer volume fraction $\varphi$ of dex-HEMA hydrogels with various DS and concentrations, before degradation.

Figure 4 shows the swelling pressure $\Pi_{sw}$ of a dex-HEMA hydrogel de-swollen in a polyethylene glycol solution after certain periods of time as a function of the polymer volume fraction $\varphi$.

Figure 5 shows the variation, as a function of degradation time t, of the constant A in the equation of Horkay et al. (see hereunder) linking the swelling pressure $\Pi_{sw}$ to the polymer volume fraction $\varphi$ in a dex-HEMA hydrogel.

Figure 6 shows the variation of the swelling pressure $\Pi_{sw}$, as a function of degradation time t, for two types of dex-HEMA hydrogels.

Figure 7 shows the variation of, the swelling pressure $\Pi_{sw}$, as a function of degradation time t, of a methacrylated dextran (hereinafter referred to as dex-MA) hydrogel during degradation by dextranase.

Figure 8 shows the variation of the osmotic pressure, as a function of time, of buffer diluted solutions of degrading diblock and triblock copolymers of lactic acid and polyethyleneglycol.

Figure 9 shows the variation of the osmotic pressure, as a function of time, of a buffer diluted solution of degrading $\gamma$-cyclodextrin.

## DETAILED DESCRIPTION OF THE INVENTION

The present invention provides the use of an *in vivo* degradable and swellable dextran hydrogel as a component of a time-controlled explosion bio-agent release system or a pulsed bio-agent delivery system comprising at least one biologically active agent and an outer lipid or polymer membrane, wherein bio-agent release or delivery begins after a predetermined time (so-called "lag time"). Depending upon the construction of the release or delivery system, i.e. in particular depending upon the selection of the particular *in vivo* degradable dextran hydrogel, the selection of the particular bio-agent, the drug loading and the intended route of administration, the lag time may vary within extremely broad ranges from about one hour to two weeks, preferably from 1 to 24 hours.

Basically, *in vivo* degradation of the degradable dextran hydrogel occurs by cleavage (i.e. usually hydrolysis) of the dextran backbone or by cleavage (i.e. usually hydrolysis) of cross-linking bonds (usually covalent bonds) within the dextran hydrogel, depending on the chemical type of the said oligomer or polymer. Whatever the degradation mechanism may be, the lag time, i.e. the time period after which the membrane ruptures, is at least partially, preferably mainly, and more preferably substantially completely controlled by the degradation rate of the said degradable dextran hydrogel. In other words, other parameters of the delivery system such as the composition and thickness of the membrane, although they may be modified in order to improve the system efficiency while using general knowledge of those skilled

in the art, are not critical to the present invention and therefore will not be discussed in detail herein. Although this parameter is not a requirement of the invention, the concentration of the degradable oligomer or polymer in the aqueous solution may be, depending on the specific nature of said oligomer or polymer and on the specific nature of the semi-permeable membrane, within a range from about 1% to about 40% by weight, preferably from about 5% to about 30% by weight.

The term "linkage" or "linker" is used herein to refer to groups or bonds that normally are formed as the result of a chemical reaction and typically are covalent linkages. Hydrolytically degradable linkages means that the linkages are degradable in water or in aqueous solutions at useful pHs, e.g. under physiological conditions, including for example blood. Enzymatically degradable linkages means that the linkage can be degraded by one or more enzymes.

For the purpose of the present invention, the *in vivo* degradable and swellable dextran hydrogels are modified dextran hydrogels which are enzymatically cleavable by dextranase. As is well known, dextran is a high molecular weight (about 15,000 to 150,000) polysaccharide containing α-glucopyranose units which may be produced from the action of *Leuconostoc mesenteroides* onto saccharose. Dextran may be chemically modified by reaction with functional α-β ethylenically unsaturated acid esters such as functional acrylates and methacrylates. The modified dextran hydrogel is a dextran modified by means of at least one ($C_{1-8}$alkyl) acrylate or methacrylate, or (hydroxy-$C_{1-8}$alkyl) acrylate or methacrylate. Methacrylated dextran (hereinafter referred to as dex-MA) may be obtained by coupling glycidyl methacrylate to dextran, as disclosed by Van Dijk et al. in Macromolecules (1995) 28:6317-6322. Dextran may also be modified by one or more ($C_{1-8}$alkyl) acrylate or methacrylate by reacting dextran with an epoxy (meth)acrylate such as the superior homologues of glycidyl acrylate or methacrylate. Within the polymer aqueous solutions or hydrogels of such (meth) acrylated dextrans, degradation occurs by cleavage of the polymer backbone, more specifically by the enzymatic action of dextranase and/or by hydrolysis of the carbonate ester link formed between the methacrylate group and the dextran molecule.

Dextran may also be modified by means of at least one (hydroxy$C_{1-8}$alkyl) acrylate or methacrylate, such as for instance hydroxyethyl methacrylate, thus leading to a structure which may be represented by the following formula:

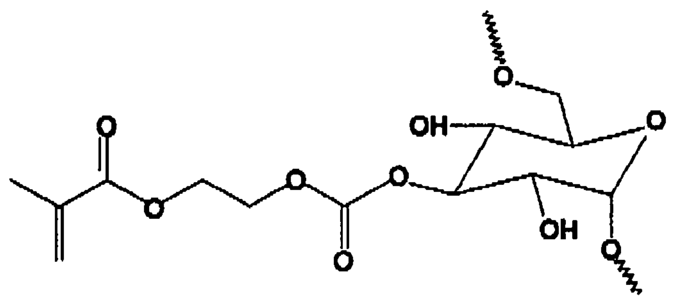

Dextran may also be modified by means of other α,β-ethylenically unsaturated entities, such as for instance acrylamides and methacrylamides, provided that that the bonds thus formed are degradable.

In polymer solutions and hydrogels from the latter modified dextran, the formation of covalent cross-linking bonds is a common feature and, hence, degradation occurs mainly by cleavage (hydrolysis) of the said cross-linking bonds within the hydrogel.

Preferably the degree of substitution (i.e. the number of (meth)acrylic groups per 100 glucopyranose residues of dextran) of the modified dextran used in this invention is between about 2 and 10.

After the chemical modification of dextran, the resulting modified dextran may be dissolved in a buffer at a suitable pH, e.g. between about 6.5 and 8.5, and the resulting aqueous solution may then be radically polymerized in the presence of a suitable soluble catalyst or catalytic system comprising, for example, N, N, N', N'-tetramethylene ethylenediamine and potassium persulfate until a hydrogel is obtained. Gelation can also be obtained by photopolymerisation in the absence or in the presence of a photoinitiator.

This dextran hydrogel is then ready to be used as a component of a time-controlled explosion bio-agent release system or a pulsed bio-agent delivery system comprising at least one biologically active agent and an outer semi-permeable lipid or polymer membrane. The term "semi-permeable", as used herein, means a membrane which is permeable to ions and water, but impermeable to the bio-agent. The term " bio-agent ", as used herein, is intended to mean any substance having biological activity such as, but not limited to, substances selected from the group consisting of therapeutic and prophylactic drugs and synthetic molecules, proteins, nucleic acids, vitamins, hormones, nutrients, aromas (fragances) and pesticides.

The therapeutic agent may be selected for its specific properties such as for instance its anti-thrombotic, anti-inflammatory, anti-proliferative or anti-microbial efficiency. The latter include for instance anti-microbial agents such as

broad spectrum antibiotics for combating clinical and sub-clinical infection, for example gentamycin, vancomycine and the like. Other suitable therapeutic agents are naturally occurring or synthetic organic or inorganic compounds well known in the art, including non-steroidal anti-inflammatory drugs, proteins and peptides (produced either by isolation from natural sources or recombinantly), hormones (for example androgenic, estrogenic and progestational hormones such as oestradiol), bone repair promoters, carbohydrates, antineoplastic agents, antiangiogenic agents, vasoactive agents, anticoagulants, immunomodulators, cytotoxic agents, antiviral agents, antibodies, neurotransmitters, oligonucleotides, lipids, plasmids, DNA and the like. Suitable therapeutically active proteins include e.g. fibroblast growth factors, epidermal growth factors, platelet-derived growth factors, macrophage-derived growth factors such as granulocyte macrophage colony stimulating factors, ciliary neurotrophic factors, tissue plasminogen activator, B cell stimulating factors, cartilage induction factor, differentiating factors, growth hormone releasing factors, human growth hormone, hepatocyte growth factors, immunoglobulins, insulin-like growth factors, interleukins, cytokines, interferons, tumor necrosis factors, nerve growth factors, endothelial growth factors, osteogenic factor extract, T cell growth factors, tumor growth inhibitors, enzymes and the like, as well as fragments thereof. Suitable diagnostic agents include conventional imaging agents (for instance as used in tomography, fluoroscopy, magnetic resonance imaging and the like) such as chelates of a transition metal (e.g. a radioactive metal selected from the group consisting of $^{99m}Tc$, $^{111}In$, $^{67}Ga$, $^{90}Y$, $^{186}Re$ and $^{188}Re$ or a non-radioactive metal selected from gadolinium, manganese and iron).

Suitable anti-microbial agents include e.g. halogenated phenols, chlorinated diphenylethers, aldehydes, alcohols such as phenoxyethanol, carboxylic acids and their derivatives, organometallic compounds such as tributyltin compounds, iodine compounds, mono- and polyamines, sulfonium and phosphonium compounds; mercapto compounds as well as their alkaline, alkaline-earth and heavy metal salts; ureas such as trihalocarbanilide, isothia- and benzisothiazolone derivatives. Suitable insecticides include natural ones, e.g. nicotine, rotenone, pyrethrum and the like, and synthetic ones like chlorinated hydrocarbons, organophosphorus compounds, biological insecticides (e.g. products derived from *Bacillus thuringiensis),* synthetic pyrethroids, organosilicon compounds, nitro-imines and nitromethylenes, Suitable fungicides include e.g. dithiocarbamates, nitrophenol derivatives, heterocyclic compounds (including thiophtalimides, imidazoles, triazines, thiadiazoles, triazoles and the like), acylalanines, phenylbenzamides and tin compounds. Suitable herbicides include e.g. trichloroacetic and aromatic carboxylic acids and their salts, substituted ureas and triazines, diphenyl ether derivatives, anilides, uraciles, nitriles and the like. Suitable fertilizers include e.g. ammonium sulphate, ammonium nitrate, ammonium phosphate and the like, and mixtures thereof.

Therapeutic agents which are advantageously delivered according to the present invention belong to all permeability and solubility classes of the Biopharmaceutical Classification System according to G. Amidon et al. in Pharm. Res. (1995) 12:413-420. As will be appreciated by those skilled in the art, these drugs belong to various therapeutic classes including β-blockers, calcium antagonists, ACE inhibitors, sympathomimetic agents, hypoglycaemic agents, contraceptives, α-blockers, diuretics, anti-hypertensive agents, antipsoriatics, bronchodilators, cortisones, anti-mycotics, salicylates, cytostatics, antibiotics, virustatics, antihistamines, UV-absorbers, chemotherapeutics, antiseptics, estrogens, scar treatment agents, antifungals, antibacterials, antifolate agents, cardiovascular agents, nutritional agents, antispasmodics, analgesics and the like.

This invention is suitable e.g. for the following therapeutic or cosmetic agents: acebutolol, acetylcysteine, acetylsalicylic acid, acyclovir, alfuzosine, alprazolam, alfacalcidol, allantoin, allopurinol, alverine, ambroxol, amikacin, amiloride, aminoacetic acid, amiodarone, amitriptyline, amlodipine, amoxicillin, ampicillin, ascorbic acid, aspartame, astemizole, atenolol, beclomethasone, benserazide, benzalkonium hydrochloride, benzocaine, benzoic acid, betamethasone, bezafibrate, biotin, biperiden, bisoprolol, bromazepam, bromhexine, bromocriptine, budesonide, bufexamac, buflomedil, buspirone, caffeine, camphor, captopril, carbamazepine, carbidopa, carboplatin, cefachlor, cefalexin, cefatroxil, cefazolin, cefixime, cefotaxime, ceftazidime, ceftriaxone, cefuroxime, cephalosporins, cetirizine, chloramphenicol, chlordiazepoxide, chlorhexidine, chlorpheniramine, chlortalidone, choline, cyclosporin, cilastatin, cimetidine, ciprofloxacin, cisapride, cisplatin, clarithromycin, clavulanic acid, clomipramine, clonazepam, clonidine, clotrimazole, codeine, cholestyramine, cromoglycic acid, cyanocobalamin, cyproterone, desogestrel, dexamethasone, dexpanthenol, dextromethorphan, dextropropoxiphen, diazepam, diclofenac, digoxin, dihydrocodeine, dihydroergotamine, dihydroergotoxin, diltiazem, diphenhydramine, dipyridamole, dipyrone, disopyramide, domperidone, dopamine, doxycycline, enalapril, ephedrine, epinephrine, ergocalciferol, ergotamine, erythromycin, estradiol, ethinylestradiol, etoposide, Eucalyptus globulus, famotidine, felodipine, fenofibrate, fenoterol, fentanyl, flavine mononucleotide, fluconazole, flunarizine, fluorouracil, fluoxetine, flurbiprofen, furosemide, gallopamil, gemfibrozil, Ginkgo biloba, glibenclamide, glipizide, clozapine, Glycyrrhiza glabra, griseofulvin, guaifenesin, haloperidol, heparin, hyaluronic acid, hydrochlorothiazide, hydrocodone, hydrocortisone, hydromorphone, ipratropium hydroxide, ibuprofen, imipenem, indomethacin, iohexol, iopamidol, isosorbide dinitrate, isosorbide mononitrate, isotretinoin, ketotifen, ketoconazole, ketoprofen, ketorolac, labetalol, lactulose, lecithin, levocamitine, levodopa, levoglutamide, levonorgestrel, levothyroxine, lidocaine, lipase, imipramine, lisinopril, loperamide, lorazepam, lovastatin, medroxyprogesterone, menthol, methotrexate, methyldopa, methylprsdni-solone, metoclopramide, metoprolol, miconazole, midazolam, minocycline, minoxidil, misoprostol, morphine, N-methylephedrine, naftidrofuryl, naproxen, neomycin, nicardipine, nicergoline, nicotinamide, nicotine, nicotinic acid, nifedipine, nimodipine, nitrazepam, nitrendipine, nizatidine, norethisterone, norfloxacin, norgestrel, nortriptyline, nystatin, ofloxacin, omeprazole, ondansetron, pancreatin, panthenol, pantothenic acid, paracetamol, paroxetine, penicillins, phenobarbital, pentoxifylline, phenoxymethylpenicillin, phenylephrine, phenylpropanolamine, phenytoin, physostigmine, piroxicam, polymyxin B, povidone iodine, pravastatin, prazepam, prazosin, prednisolone, prednisone, bromocriptine, propafenone, propranolol, proxyphylline, pseudoephedrine, pyridoxine, quinidine, ramipril, ranitidine, reserpine, retinol, riboflavin, rifampicin, rutoside, saccharin, salbutamol, salcatonin, salicylic acid, simvastatin, somatotropin, sotalol, spironolactone, sucralfate, sulbactam, sulfamethoxazole, sulfasalazine, sulpiride, tamoxifen, tegafur, teprenone, terazosin, terbutaline, terfenadine, tetracaine, tetracycline, theophylline, thiamine, ticlopidine, timolol, tranexamic acid, tretinoin, triamcinolone acetonide, triamterene, triazolam, trimethoprim, troxerutin, uracil, valproic acid, verapamil, folinic acid, zidovudine, zopiclone, enantiomers thereof, organic and inorganic salts thereof, hydrates thereof and mixtures thereof, in particular mixtures in synergistic proportions.

Other bio-agents suitable for the purpose of the invention are vitamins, include those of the A group, of the B group (which means, besides B1, B2, B6 and B12, also compounds with vitamin B properties such as adenine, choline, pantothenic acid, biotin, adenylic acid, folic acid, orotic acid, pangamic acid, carnitine, p-aminobenzoic acid, myo-inositol and lipoic acid), vitamin C, vitamins of the D group, E group, F group, H group, I and J groups, K group and P group.

This invention is also suitable for therapeutic agents (drugs) having a water-solubility as low as about 0.2 $\mu$g/ml. Examples of such drugs include for instance hydrochlorothiazide, nimodipine, flufenamic acid, mefenamic acid, bendroflumethiazide, benzthiazide, ethacrinic acid, nitrendipine and diaminopyrimidines. Suitable examples of such poorly soluble diaminopyrimidines include, without limitation, 2,4-diamino-5-(3,4,5-trimethaxybenzyl) pyrimidine (trimethoprim), 2,4-diamino-5-(3,4-dimethoxy-benzyl) pyrimidine (diaveridine), 2,4 diamino-5-(3,4,6-trimethoxybenzyl) pyrimidine, 2,4-diamino-5-(2-methyl-4,5-dimethoxybenzyl) pyrimidine (ormeto-prim), 2,4-diamino-5-(3,4-dimethoxy-5-bromobenzyl) pyrimidine, 2,4-diamino-5-(4-chloro-phenyl)-6-ethylpyrimidine (pyrimetha-mine), and analogues thereof.

This invention is suitable for said therapeutic agents (drugs) which further comprise one or more pharmaceutically acceptable excipients, such as emulsifiers or surface-active agents, thickening agents, gelling agents or other additives, and wherein the drug loading (i.e. the proportion of the drug in the formulation) may vary through a wide range from about 5% by weight to about 95% by weight.

Emulsifiers or surface-active agents suitable for therapeutic agents formulations include water-soluble natural soaps and water-soluble synthetic surface-active agents. Suitable soaps include alkaline or alkaline-earth metal salts, unsubstituted or substituted ammonium salts of higher, preferably saturated, fatty acids ($C_{10}$-$C_{22}$), e.g. the sodium or potassium salts of oleic or stearic acid, or of natural fatty acid mixtures obtainable form coconut oil, palm oil or tallow oil. Synthetic surface-active agents (surfactants) include anionic, cationic and non-ionic surfactants, e.g. sodium or calcium salts of polyacrylic acid; sulphonated benzimidazole derivatives preferably containing 8 to 22 carbon atoms; alkylarylsulphonates; and fatty sulphonates or sulphates, usually in the form of alkaline or alkaline-earth metal salts, unsubstituted ammonium salts or ammonium salts substituted with an alkyl or acyl radical having from 8 to 22 carbon atoms, e.g. the sodium or calcium salt of lignosulphonic acid or dodecylsulphonic acid or a mixture of fatty alcohol sulphates obtained from natural fatty acids, alkaline or alkaline-earth metal salts of sulphuric or sulphonic acid esters (such as sodium lauryl sulphate) and sulphonic acids of fatty alcohol/ethylene oxide adducts. Examples of alkylarylsulphonates are the sodium, calcium or alcanolamine salts of dodecylbenzene sulphonic acid or dibutyl-naphtalenesulphonic acid or a naphtalene-sulphonic acid/formaldehyde condensation product. Also suitable are the corresponding phosphates, e.g. salts of phosphoric acid ester and an adduct of p-nonylphenol with ethylene and/or propylene oxide) and the like.

Suitable emulsifiers further include partial esters of fatty acids (e.g. lauric, palmitic, stearic or oleic) or hexitol anhydrides (e.g., hexitans and hexides) derived from sorbitol, such as commercially available polysorbates. Other emulsifiers which may be used include adducts of polyoxyethylene chains (1 to 40 moles ethylene oxide) with non-esterified hydroxyl groups of the above partial esters, such as the surfactant commercially available under the trade name Tween 60 from ICI Americas Inc.; and the poly(oxyethylene)/poly(oxypropylene) materials marketed by BASF under the trade name Pluronic.

Suitable structure-forming, thickening or gel-forming agents for the bio-agents of the invention include highly dispersed silicic acid, such as the product commercially available under the trade name Aerosil; bentonites; tetraalkyl ammonium salts of montmorillonites (e.g. products commercially available under the trade name Bentone) wherein each of the alkyl groups may contain from 1 to 20 carbon atoms; cetostearyl alcohol and modified castor oil products (e.g. a product commercially available under the trade name Antisettle).

Gelling agents which may be included into the bio-agent formulation of the present invention include cellulose derivatives such as carboxymethylcellulose, cellulose acetate and the like; natural gums such as arabic gum, xanthum gum, tragacanth gum, guar gum and the like; gelatin; silicium dioxide; synthetic polymers such as carbomers, and mixtures thereof. Gelatin and modified celluloses represent a preferred class of gelling agents.

Other optional excipients which may be present in the bio-agent formulation of the present invention include additives such as magnesium oxide; azo dyes; organic and inorganic pigments such as titanium dioxide; UV-absorbers;

stabilisers; odor masking agents; viscosity enhancers; antioxidants such as, for example, ascorbyl palmitate, sodium bisulfite, sodium metabisulfite and the like, and mixtures thereof; preservatives such as, for example, potassium sorbate, sodium benzoate, sorbic acid, propyl gallate, benzylalcohol, methyl paraben, propyl paraben and the like; sequestering agents such as ethylenediamine tetraacetic acid; flavoring agents such as natural vanillin; buffers such as citric acid or acetic acid; extenders or bulking agents such as silicates, diatomaceous earth, magnesium oxide or aluminum oxide; densification agents such as magnesium salts; and mixtures thereof.

Time-controlled explosion bio-agent release systems and pulsed bio-agent delivery systems according to this invention may take different forms in terms of shape, size, composition and number of layers, including embodiments such as contemplated hereinbefore. For instance, the pulsed delivery system may be in the form of beads or granules comprising a core covered with one or more outer layers. They usually comprise at least (i) an outer semi-permeable membrane wherein bio-agent release or delivery is caused by explosion of the said membrane and begins after a certain lag time and at least (ii) a core comprising a bio-agent and a swelling agent, and are further characterised in that the said swelling agent is a degradable polymer aqueous solution or hydrogel, preferably of the type wherein degradation occurs by cleavage of the polymer backbone or by cleavage of cross-linking bonds within the hydrogel. Although a membrane and a core such as above defined are the main requirements of such systems, more elaborate structures such as including additional intermediate layers comprising further excipients (such as defined hereinbefore) cannot be excluded.

In a suitable working embodiment of the invention, the bio-agent is present in the core in the form of micro- or nanoparticles. The bio-agent may also be intimately admixed with the swelling agent.

The present invention relates to releasing the bio-agent as a single pulse. In specific cases, it may be beneficial to provide multiple pulsed delivery or multiple explosion release of the bio-agent. This may be effected by providing the core of the delivery system with a mixture of at least two swelling agents having different degradation rates such as to provide two or more different lag times for the bio-agent(s). In a specific embodiment for this purpose, the core of the delivery system comprises at least a first population of micro- or nanoparticles including a first bioagent and a first swelling agent and a second population of micro- or nanoparticles including a second bioagent and a second swelling agent, so that the first bioagent is released or delivered after a first lag time and the second bioagent is released or delivered after a second lag time, the said second lag time being substantially different from the said first lag time. In this embodiment, the first bio-agent may be different from or the same as the second bio-agent, thus providing additional flexibility for the biological, e.g. therapeutic or prophylactic, treatment. As previously mentioned, each of the first and subsequent lag times may vary within very broad ranges from about one hour to two weeks.

The pulsed delivery systems of the present invention are suitable for a number of different ways of administration of therapeutic agents such as, but not limited to, oral administration, parenteral administration, subcutaneous administration, vaccination and the like.

Although the swelling behaviour of polymer networks has been the subject of numerous investigations, according to our knowledge the variation of the swelling pressure in degrading gel systems has not previously been the subject of significant investigations. Therefore it should be understood that this invention may be performed in a number of different ways without departing from its original concept. The following examples provide a selection of a few appropriate working embodiments for this invention.

## EXAMPLE 1 - Dex-HEMA preparation and characterization

Dex-HEMA batches were prepared and characterized according to the method described by Van Dijk et al. (cited *supra),* using dextran (commercially available from Fluka, obtained from *Leuconostoc ssp.)* with a molecular weight $M_n$=19 000. The degree of substitution (hereinafter DS) of Dex-HEMA was determined by proton nuclear magnetic resonance spectroscopy (H-NMR) in $D_2O$ with a Gemini 300 spectrometer (Varian). The DS of samples used in the following examples were 2.9, 5.0 and 7.5, respectively.

## EXAMPLE 2 - preparation and degradation of dex-HEMA hydrogels

Dex-HEMA gels were made by radical polymerization of aqueous dex-HEMA solutions by first dissolving dex-HEMA in a phosphate buffer (10 mM $Na_2HPO_4$, 0.02% sodium azide, adjusted with 1 N hydrochloric acid to pH 7.0). The polymerization reagents were N,N,N',N'-tetramethylene ethylenediamine (50 $\mu$l of a 20% volume/volume solution in deoxygenated phosphate buffer, pH 8.5, added to 1 g polymer solution) and potassium persulfate (90 $\mu$l of a 50 mg/ml solution in deoxygenated phosphate buffer). The reactor were coated with polyethylene glycol (hereinafter PEG) (molecular weight 20,000; 10% solution in phosphate buffer) in order to reduce adhesion. Gelation required about 1 hour at 23°C. The hydrogel samples used in the following rheological measurements were made in cylindrical molds (diameter 23 mm, height 2 mm).

For the other experiments, gels were prepared in 2.5 ml polypropylene syringes (diameter 8.5 mm) from which

the heads were sawn. After gelation the gel samples were removed from the syringe and cut with a thin wire. Degradation was studied in phosphate buffer (pH 7) at 37 °C. Throughout the following examples, the dex-HEMA concentration (expressed in weight%) refers to the concentration at which cross-links were introduced.

EXAMPLE 3 - osmotic de-swelling of dex-HEMA hydrogels

Osmotic deswelling measurements were performed on dex-HEMA gels using the method described by Horkay et al. in Macromolecules (1982) 15:1306-1310. Gel specimens were surrounded by a semi-permeable membrane (Medicell dialysis bags, $M_w$ between 12 000 and 14 000). Similar dialysis bags were used in the purification step of the synthesis of dex-HEMA in example 1 above.

After different degradation times, gel samples were equilibrated with PEG-solutions at 4°C. PEG (available from Merck, $M_w$=20,000) was dissolved in citrate buffer (9.44 g/l $Na_2HPO_4$; 10.3 g/l citric acid and 0.2 g/l $NaN_3$, pH 4.4). The PEG concentration was varied in the range from 0 to 12.5 g/100 ml. It was verified that further degradation of the dex-HEMA gels did not occur during the osmotic de-swelling measurements. Equilibrium swelling was attained within 7 days. The reversibility of the swelling process was checked.

At equilibrium, the swelling pressure of the gel is equal to the osmotic pressure of the PEG-solution. The osmotic pressure of the PEG-solution was calculated from the equation disclosed by Nichol et al. in Biochem J. (1967) 102: 407-416 as follows:

$$\Pi_{PEG} = \left[\frac{1}{M_n} + A_2\, c + A_3\, c^2\right] . cRT \times 10 \qquad (1)$$

where R is the gas constant, T is the absolute temperature, c is the PEG concentration (in g/100 ml),and $A_2$ and $A_3$ are the second and third virial coefficient, respectively. According to the data reported by Edmond et al. in Biochem J. (1968) 109:569-576 for PEG ($M_n$ = 20,000), $A_2$=2.59x$10^{-5}$ (mol.$10^2$ ml)/$g^2$ and $A_3$=1.35x$10^{-6}$ (mol.$10^4$ $ml^2$)/$g^3$.

The dex-HEMA concentration of the gels was calculated using the relationship

$$c = \frac{w_{dex-HEMA}}{\left[\left(w_{dex-HEMA} \times v_1\right) + \left(\frac{w_e - w_{dex-HEMA}}{\rho}\right)\right]} \times 100 \qquad (2)$$

where $w_e$ is the weight of the dex-HEMA gel, $w_{dex\text{-}HEMA}$ is the weight of dex-HEMA determined gravimetrically after drying the gel in a vacuum oven at 50°C, ρ is the density of the buffer and $v_1$ is the specific volume of the dex-HEMA ($v_1$ =0.72 ml/g) as disclosed by De Smedt et al. in Macromolecules (1995) 28:5082-5088. The polymer volume fraction ( ) of the gels was calculated from the concentration of dex-HEMA and $v_1$.

EXAMPLE 4 - mechanical characterization of degrading dex-HEMA hydrogels

Rheological measurements were performed using an AR1000-N controlled stress rheometer (available from TA-Instruments). In order to avoid slippage, the acrylic top plate was covered by sandpaper (diameter 2 cm). The bottom plate was replaced with a Plexiglas® plate with a roughened surface. Measurements were done in oscillation mode at 1 Hz in the linear visco-elastic region of these gels by applying a constant strain of 0.5%. After measurement, the hydrogel samples were transferred into phosphate buffer and stored at 37°C. Further details of this method are provided by Meyvis et al. in J. Rheol. (1999) 43:933-950.

EXAMPLE 5 - determination of free dextran chains in dex-HEMA gels

The concentration of free dextran in the dex-HEMA hydrogels of example 2 was determined from a release experiment performed in phosphate buffer at 37°C. The amount of dextran chains in the solution was measured by gel permeation chromatography (GPC) in a system consisting of a high pressure pump (Waters M510), an injector (Waters U6K) and a differential refractometer (Waters 410). 250 μl of each sample was injected and a flow rate of 0.5 ml/min was applied. The dex-HEMA concentration was calculated from the height of the peak using a calibration curve (between 0 and 2.5 mg/ml) obtained for the corresponding dex-HEMA.

RESULTS AND DISCUSSION

Figure 1 shows the amount of dextran released from different dex-HEMA gels as a function of degradation time: first the sol fraction (unreacted dex-HEMA chains) leaves the gel, this feature being independent of the degradation process. In the second region (delay region) a relatively small amount of dextran is released. Finally, when the majority of cross-links are cleaved, liberation of dextran chains is significantly enhanced.

Figure 2 shows the elastic modulus G' as a function of the degradation time and exhibits a continuous decrease during the degradation process. The decrease of G' is significantly slower in gels having higher dex-HEMA concentration or higher DS. Since G' is proportional to the cross-link density, this finding indicates that degradation is slower in densely cross-linked gels.

In order to elucidate the effect of degradation on thermodynamic properties we measured the swelling pressure $\Pi_{sw}$ at different stages of degradation. The swelling pressure $\Pi_{sw}$ of a non-ionic gel can be described as the sum an osmotic pressure $\Pi_{osm}$ that expands the network and an elastic pressure $\Pi_{el}$ that acts against expansion:

$$\Pi_{sw} = \Pi_{osm} + \Pi_{el} \quad (3)$$

Figure 3 shows the swelling pressure as a function of the polymer volume fraction for different undegraded dex-HEMA hydrogels. The continuous curves are the least squares fits of the swelling pressure data according to Horkay et al. (citedsupra):

$$\Pi_{sw} = A\,\varphi^{n} - A\,\varphi_e^{n-1/3}\,\varphi^{1/3} \quad (4)$$

where *A* is a constant depending on the particular polymer-solvent system, $\varphi_e$ and $\varphi$ are the volume fraction of the polymer in equilibrium with pure buffer and PEG solutions, respectively. For the exponent n, the scaling theory (P. G. De Gennes, Scaling concept in polymer physics, published 1979) predicts n = 2.31 (good solvent condition) and n=3.0 ($\Theta$-solvent condition). The values of A and n obtained from the fits to equation 4 are listed in Table 1 hereinafter. As expected A depends on the chemical composition of the network. The value of n is close to that predicted for good solvent condition.

The effect of degradation on the swelling pressure was studied on the sample having the shortest degradation time (Dex-HEMA DS2.9; 25%). Figure 4 shows the swelling pressure as a function of the polymer volume fraction measured at different stages of degradation (up to 30 days). The $\Pi_{sw}$ versus $\varphi$ curves are gradually shifted to the left as the gel degrades.

The parameters obtained from the fits to equation 4 at different degradation times (Table 1, Figure 5) indicate that neither *A* or n varies notably in the first 15 days of degradation. It can also be seen that after 30 days (i.e., when the network is completely liquified) the value of *A* significantly increases.

The dashed line in Figure 4 shows the situation that occurs when the gel is surrounded by a rigid semi-permeable membrane. During degradation, $\Pi_{sw}$ increases from 0 kPa (swelling pressure of the fully swollen undegraded gel) to 49 kPa (swelling pressure of the totally degraded dex-HEMA gel). The latter is the hydrostatic pressure required to maintain the initial concentration ($\varphi$=0.112) of the gel during the degradation process.

The above results indicate that the degradation rate strongly depends on the initial dex-HEMA concentration and DS. The variation of the swelling pressure at each stage of degradation is satisfactorily described by equation (4). In the earlier phase of the degradation process, the swelling pressure gradually increases because of the decrease of the elastic pressure. Towards the end of the degradation process, a pronounced increase in the swelling pressure is observed and is accompanied by a sudden increase in the amount of dextran released from the gel.

In drug delivery systems, osmotic pressure can cause rupture of the membrane surrounding the hydrogel particle. Consequently, the detailed knowledge of the variation of the swelling pressure during the degradation process, such as investigated by the above methodology, is essential to design systems based on degradable hydrogels that have a swelling pressure profile tailored for pulsed delivery of drugs.

Table 1

| sample | degradation time (days) | volume fraction [a] | A (kPa) | n |
|---|---|---|---|---|
| DS 7.5; 20% | 0 | 0.1150 | 4554 | 2.35 |

(continued)

| sample | degradation time (days) | volume fraction [a] | A (kPa) | n |
|---|---|---|---|---|
| DS 5.0; 20% | 0 | 0.1391 | 5767 | 2.33 |
| DS 2.9; 20% | 0 | 0.1547 | 3587 | 2.33 |
| DS 2.9; 30% | 0 | 0.1688 | 3231 | 2.36 |
| DS2.9; 25% | 0 | 0.1128 | 5562 | 2.36 |
| DS2.9; 25% | 3 | 0.0919 | 5563 | 2.34 |
| DS2.9; 25% | 6 | 0.0745 | 5554 | 2.35 |
| DS2.9; 25% | 10 | 0.0598 | 5487 | 2.34 |
| DS2.9; 25% | 15 | 0.0493 | 5337 | 2.30 |
| DS2.9; 25% | 30 | < 0.01 | 7920 | 2.33 |

[a] volume fraction at equilibrium swelling

EXAMPLE 6

Dex-HEMA/dextran hydrogels were made as in Example 2. The solutions were prepared by dissolving dex-HEMA and dextran in phosphate buffer. The dextran (from *Leuconostoc mesenteroides,* Merck, $M_n$ = 19,000) was the same as used in the dex-HEMA synthesis. The swelling pressure of gels prepared in the presence of free dextran chains was determined by a home-built swelling pressure osmometer. This device consists of a calibrated transducer (Honeywell), a sample chamber (volume: 4.2 mL) and a buffer chamber (filled with 15 mL phosphate buffer at pH 7.0); the chambers are separated by a semi-permeable membrane (Medicell, $M_w$ cut-off between 12,000 and 14,000) supported by a porous Bekipor® frame which is further supported by a Teflon perforated cylinder. The membrane is permeable to small molecules (water and ions) but impermeable to large dextran molecules. The apparatus measures $_{sw}$ up to 7 atmospheres. $_{sw}$ measurements were performed on gels made in the sample chamber 12 hours after preparation, i.e. before substantial degradation occurred. Measurements were made at 4°C, thus preventing hydrolysis of the dex-HEMA/dextran hydrogels. The reproducibility of the swelling pressure measurements was found to be better than ± 2%. In figure 6 are presented swelling pressure data ($_{sw}$, expressed in kPa) versus degradation time plots obtained from swelling pressure measurements of a dex-HEMA/dextran gel with DS 2.9 at a concentration of 25% by weight (left, showing a critical time of about 18 days) and a dex-HEMA/dextran gel with DS 5 at a concentration of 20% by weight (right, showing a critical time of about 36 days).

EXAMPLE 7 - swelling pressure of dex-MA hydrogels

Methacrylated dextran (Dex-MA) with a DS = 4.0 was prepared according to the method disclosed by Van Dijk et al. in Macromolecules (1995) 28:6317-6322. Hydrogels were prepared by radical polymerisation of an aqueous solution of dex-MA, said solutions being prepared by dissolving the dex-MA thus obtained in phosphate buffer (PB) (10 mM $Na_2HPO_4$, 0.02% sodium azide, adjusted with 1 N hydrochloric acid to pH 7.0) at a concentration of 20% by weight. Prior to addition of the gelation reagents, the enzyme solution (D-1508 Sigma; diluted to 10 U/ml in 10 mM PB pH 7.0; one unit delivers 1 μmole of isomaltose per minute at pH 6 at 37°C) was added to the dex-MA solution (cooled to 4°C) in such a way that its final concentration corresponds to 0.25 unit per gram of gel.. Gelation started after adding 50 μl N,N,N',N'-tetramethylene-ethylenediamine (commercially available from Fluka; 20% by volume in deoxygenated PB, pH adjusted to 8.5 with hydrochloric acid) per gram, followed under stirring by 90 μl potassium persulfate (commercially available from Fluka; 50 mg/ml in deoxygenated PB) per gram. Gels were immersed directly in the membrane osmometer. The swelling pressure of the enzymatically degrading Dex-MA hydrogels was determined by the swelling pressure osmometer of example 6. The data of swelling pressure measurements are shown in figure 7.

EXAMPLE 8 - osmotic pressure changes of lactic acid-polyethylene glycol block copolymers

The change in osmotic pressure of two different degrading polymer solutions was measured by using freezing - point osmometry (using the Advanced Micro-osmometer Model 3300, commercially available from Advanced Instruments, Inc.). Chemical hydrolysis of a (lactic acid-b-polyethylene glycol) diblock copolymer (with block molecular weights of 456 and 2000 respectively) and a (lactic acid-b-polyethylene glycol-b-lactic acid) triblock copolymer (with block molecular weights of 231, 1450 and 231 respectively) was followed as a function of time. Therefore 5% by weight solutions were made of each polymer in N-2-hydroxyethylpiperazine-N'-2-ethanesulfonic acid (HEPES) buffer (50mM, pH 7.2). Both copolymer solutions were degraded at 37°C. 20μL samples were taken at well defined times and the osmotic

pressure (in mOsm) was measured immediately. Measurement results are shown in figure 8.

Reference EXAMPLE 9- osmotic pressure changes of -cyclodextrin

The change in osmotic pressure of a -cyclodextrin solution was measured as in example 8. Enzymatic hydrolysis of -cyclodextrin with muco-amylase (0.06 mg/mL, corresponding to 1 U/mL) was followed as a function of time. Therefore a 5% by weight solution of -cyclodextrin was made in HEPES-buffer (50mM, pH of 7.2) and then degraded at 37°C. 20μL samples were taken at well defined times and the osmotic pressure (in mOsm) was measured immediately. Measurement results are shown in figure 9.

### Claims

1. Use of an *in vivo* degradable and swellable dextran hydrogel, wherein said dextran hydrogel is a dextran modified by means of at least one ($C_{1-8}$ alkyl) acrylate or methacrylate and wherein *in vivo* degradation occurs by cleavage of the dextran backbone and/or by cleavage of cross-linking bonds within the dextran hydrogel, as a swellable component of a time-controlled explosion bio-agent release system or a pulsed bio-agent delivery system comprising at least one biologically active agent and an outer lipid or polymer membrane which is permeable to ions and water but impermeable to the bio-agent, wherein bio-agent release or delivery begins after a lag time ranging from 1 hour to 2 weeks.

2. Use of an *in vivo* degradable and swellable dextran hydrogel, wherein said dextran hydrogel is a dextran modified by means of at least one (hydroxy-$C_{1-8}$ alkyl) acrylate or methacrylate and wherein degradation occurs by cleavage of cross-linking bonds within the dextran hydrogel, as a swellable component of a time-controlled explosion bio-agent release system or a pulsed bio-agent delivery system comprising at least one biologically active agent and an outer lipid or polymer membrane which is permeable to ions and water but impermeable to the bio-agent, wherein bio-agent release or delivery begins after a lag time ranging from 1 hour to 2 weeks.

3. Use according to claim 1 or claim 2, wherein said bio-agent is selected from the group consisting of therapeutic and prophylactic drugs and synthetic molecules, proteins, nucleic acids, vitamins, hormones, nutrients, aromas, anti-microbial agents, insecticides, fungicides, herbicides, fertilisers and pesticides.

4. Use according to claim 3, wherein said bio-agent is a therapeutic agent selected from the group consisting of non-steroidal anti-inflammatory drugs, bone repair promoters, carbohydrates, antineoplastic agents, antiangiogenic agents, vasoactive agents, anticoagulants, immunomodulators, cytotoxic agents, antiviral agents, and neurotransmitters.

5. Use according to claim 3, wherein said bio-agent is a therapeutic active protein selected from the group consisting of fibroblast growth factors, epidermal growth factors, platelet-derived growth factors, macrophage-derived growth factors such as granulocyte macrophage colony stimulating factors, ciliary neurotrophic factors, tissue plasminogen activator, B cell stimulating factors, cartilage induction factor, differentiating factors, growth hormone releasing factors, human growth hormone, hepatocyte growth factors, immunoglobulins, insulin-like growth factors, interleukins, cytokines, interferons, tumor necrosis factors, nerve growth factors, endothelial growth factors, osteogenic factor extract, T cell growth factors, tumor growth inhibitors, and fragments thereof.

6. Use according to claim 3, wherein said bio-agent is an anti-microbial agent selected from the group consisting of halogenated phenols, chlorinated diphenylethers, aldehydes, alcohols, carboxylic acids and derivatives thereof, organometallic compounds, iodine compounds, mono- and polyamines, sulfonium and phosphonium compounds ; mercapto compounds and alkaline, alkaline-earth or heavy metal salts thereof; ureas ; isothia- and benzisothiazolone derivatives.

7. Use according to claim 3, wherein said bio-agent is a therapeutic agent selected from the group consisting of acebutolol, acetylcysteine, acetylsalicylic acid, acyclovir, alfuzosine, alprazolam, alfacalcidol, allantoin, allopurinol, alverine, ambroxol, amikacin, amiloride, aminoacetic acid, amiodarone, amitriptyline, amlodipine, amoxicillin, ampicillin, ascorbic acid, aspartame, astemizole, atenolol, beclomethasone, benserazide, benzalkonium hydrochloride, benzocaine, benzoic acid, betamethasone, bezafibrate, biotin, biperiden, bisoprolol, bromazepam, bromhexine, bromocriptine, budesonide, bufexamac, buflomedil, buspirone, caffeine, camphor, captopril, carbamazepine, carbidopa, carboplatin, cefachlor, cefalexin, cefatroxil, cefazolin, cefixime, cefotaxime, ceftazidime, ceftriaxone, cefuroxime, cephalosporins, cetirizine, chloramphenicol, chlordiazepoxide, chlorhexidine, chlorpheniramine, chlortalidone, choline, cyclosporin, cilastatin, cimetidine, ciprofloxacin, cisapride, cisplatin, clarithromycin, clavulanic acid, clomipramine, clonazepam, clonidine, clotrimazole, codeine, cholestyramine, cromoglycic acid, cyanocobalamin, cyproterone, desogestrel, dexamethasone, dexpanthenol, dextromethorphan, dextropropoxiphen, diazepam, diclofenac, digoxin, dihydrocodeine, dihydroergotamine, dihydroergotoxin, diltiazem, diphenhydramine, dipyridamole, dipyrone, disopyramide, domperidone, dopamine, doxycycline, enalapril, ephedrine, epinephrine, ergocalciferol, ergotamine, erythromycin, estradiol, ethinylestradiol, etoposide, Eucalyptus globulus, famotidine, felodipine, fenofibrate, fenoterol, fentanyl, flavine mononucleotide, fluconazole, flunarizine, fluorouracil, fluoxetine, flurbiprofen, furosemide, gallopamil, gemfibrozil, Ginkgo biloba, glibenclamide, glipizide, clozapine, Glycyrrhiza glabra, griseofulvin, guaifenesin, haloperidol, heparin, hyaluronic acid, hydrochlorothiazide, hydrocodone, hydrocortisone, hydromorphone, ipratropium hydroxide, ibuprofen, imipenem, indomethacin, iohexol, iopamidol, isosorbide dinitrate, isosorbide mononitrate, isotretinoin, ketotifen, ketoconazole, ketoprofen, ketorolac, labetalol, lactulose, lecithin, levocarnitine, levodopa, levoglutamide, levonorgestrel, levothyroxine, lidocaine, lipase, imipramine, lisinopril, loperamide, lorazepam, lovastatin, medroxyprogesterone, menthol, methotrexate, methyldopa, methylprednisolone, metoclopramide, metoprolol, miconazole, midazolam, minocycline, minoxidil, misoprostol, morphine, N-methylephedrine, naftidrofuryl, naproxen, neomycin, nicardipine, nicergoline, nicotinamide, nicotine, nicotinic acid, nifedipine, nimodipine, nitrazepam, nitrendipine, nizatidine, norethisterone, norfloxacin, norgestrel, nortriptyline, nystatin, ofloxacin, omeprazole, ondansetron, pancreatin, panthenol, pantothenic acid, paracetamol, paroxetine, penicillins, phenobarbital, pentoxifylline, phenoxymethylpenicillin, phenylephrine, phenylpropanolamine, phenytoin, physostigmine, piroxicam, polymyxin B, povidone iodine, pravastatin, prazepam, prazosin, prednisolone, prednisone, bromocriptine, propafenone, propranolol, proxyphylline, pseudoephedrine, pyridoxine, quinidine, ramipril, ranitidine, reserpine, retinol, riboflavin, rifampicin, rutoside, saccharin, salbutamol, salcatonin, salicylic acid, simvastatin, somatotropin, sotalol, spironolactone, sucraffate, sulbactam, sulfamethoxazole, sulfasalazine, sulpiride, tamoxifen, tegafur, teprenone, terazosin, terbutaline, terfenadine, tetracaine, tetracycline, theophylline, thiamine, ticlopidine, timolol, tranexamic acid, tretinoin, triamcinolone acetonide, triamterene, triazolam, trimethoprim, troxerutin, uracil, valproic acid, verapamil, folinic acid, zidovudine, zopiclone, enantiomers thereof, organic and inorganic salts thereof, hydrates thereof and mixtures thereof, hydrochlorothiazide, flufenamic acid, mefenamic acid, bendroflumethiazide, benzthiazide, ethacrinic acid and diaminopyrimidines.

**8.** Use according to claims 4-7, wherein said bio-agent is a therapeutic drug and wherein the drug loading is from 5% to 95% by weight and wherein said system further comprises one or more pharmaceutically acceptable excipients.

## Patentansprüche

**1.** Verwendung eines *in vivo* abbaubaren und quellbaren Dextran-Hydrogels, wobei das Dextran-Hydrogel ein Dextran ist, das mit Hilfe von mindestens einem ($C_{1-8}$-Alkyl)acrylat oder -methacrylat modifiziert wurde, und wobei der *in vivo*-Abbau durch Spaltung des Dextran-Rückgrats und/oder durch Spaltung vernetzender Bindungen innerhalb des Dextran-Hydrogels erfolgt, als ein quellbarer Bestandteil eines Systems zur zeitgesteuerten Freisetzung biologisch wirksamer Stoffe oder eines Systems zur stoßweisen Abgabe biologisch wirksamer Stoffe, umfassend mindestens einen biologischen Wirkstoff und eine äußere Lipid- oder Polymermembran, die für Ionen und Wasser durchlässig, für den biologisch wirksamen Stoff jedoch undurchlässig ist, wobei die Freisetzung oder Abgabe biologisch wirksamer Stoffe nach einer Verzögerungszeit, die zwischen 1 Stunde und 2 Wochen beträgt, beginnt.

**2.** Verwendung eines *in vivo* abbaubaren und quellbaren Dextran-Hydrogels, wobei das Dextran-Hydrogel ein Dextran ist, das mit mindestens einem (Hydroxy-$C_{1-8}$-alkyl)acrylat oder -methacrylat modifiziert wurde und wobei der Abbau durch Spaltung vernetzender Bindungen innerhalb des Dextran-Hydrogels erfolgt, als ein quellbarer Bestandteil eines Systems zur zeitgesteuerten Freisetzung biologisch wirksamer Stoffe oder eines Systems zur stoßweisen Abgabe biologisch wirksamer Stoffe, umfassend mindestens einen biologischen Wirkstoff und eine äußere Lipid- oder Polymermembran, die für Ionen und Wasser durchlässig, für den biologisch wirksamen Stoff jedoch undurchlässig ist, wobei die Freisetzung oder Abgabe biologisch wirksamer Stoffe nach einer Verzögerungszeit, die zwischen 1 Stunde und 2 Wochen beträgt, beginnt.

**3.** Verwendung nach Anspruch 1 oder Anspruch 2, wobei der biologisch wirksame Stoff ausgewählt ist aus der Gruppe, bestehend aus Therapeutika und prophylaktischen Mitteln und synthetischen Molekülen, Proteinen, Nukleinsäuren, Vitaminen, Hormonen, Nährstoffen, Aromastoffen, antimikrobiellen Mitteln, Insektiziden, Fungiziden, Herbiziden, Düngemitteln und Pestiziden.

4. Verwendung nach Anspruch 3, wobei der biologisch wirksame Stoff ein Therapeutikum ist, das ausgewählt ist aus der Gruppe, bestehend aus nicht-steroiden, entzündungshemmenden Arzneimitteln, Promotoren der Knochenreparatur, Kohlenhydraten, antineoplastischen Mitteln, antiangiogenen Mitteln, vasoaktiven Mitteln, Antikoagulantien, Immunmodulatoren, cytotoxischen Mitteln, antiviralen Mitteln und Neurotransmittern.

5. Verwendung nach Anspruch 3, wobei der biologisch wirksame Stoff ein therapeutisch wirksames Protein ist, das ausgewählt ist aus der Gruppe, bestehend aus Fibrboblastenwachstumsfaktoren, epidermalen Wachstumsfaktoren, Platelet-derived Growth Factors, Macrophage-derived Growth Factors, wie beispielsweise Granulozyten-Makrophagen-Kolonie-stimulierenden Faktoren, ziliären, neurotrophen Faktoren, Gewebe-Plasminogenaktivator, B-Zellen stimulierenden Faktoren, Knorpelinduktionsfaktor, Differenzierungsfaktoren, Wachstumshormone freisetzenden Faktoren, humanen Wachstumshormonen, Hepatozyten-Wachstumsfaktoren, Immunglobulinen, insulinähnlichen Wachstumsfaktoren, Interleukinen, Cytokinen, Interferonen, Tumornekrosefaktoren, Nervenwachstumsfaktoren, endothelialen Wachstumsfaktoren, Extrakt des osteogenen Faktors, T-Zell-Wachstumsfaktoren, Inhibitoren des Wachstums von Tumoren und deren Fragmenten.

6. Verwendung nach Anspruch 3, wobei der biologisch wirksame Stoff ein antimikrobielles Mittel ist, das ausgewählt ist aus der Gruppe, bestehend aus halogenierten Phenolen, chlorierten Diphenylethern, Aldehyden, Alkoholen, Carbonsäuren und deren Derivate, organometallischen Verbindungen, Iodverbindungen, Mono- und Polyaminen, Sulfonium- und Phosphoniumverbindungen; Mercaptoverbindungen sowie deren Alkali-, Erdalkali- und Schwermetallsalze; Harnstoffe; Isothia- und Benzisothiazolonderivaten.

7. Verwendung nach Anspruch 3, wobei der biologisch wirksame Stoff ein Therapeutikum ist, das ausgewählt ist aus der Gruppe, bestehend aus Acebutolol, Acetylcystein, Acetylsalicylsäure, Acyclovir, Alfuzosin, Alprazolam, Alfacalcidol, Allantoin, Allopurinol, Alverin, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkoniumhydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefalexin, Cefatroxil, Cefazolin, Cefixim, Cefotaxim, Ceftazidim, Ceftriaxon, Cefizroxim, Cephalosporine, Cetirizin, Chloramphenicol, Chlordiazepoxid, Chlorhexidin, Chlorpheniramin, Chlortalidon, Cholin, Cyclosporin, Cilastatin, Cimetidin, Ciprofloxacin, Cisaprid, Cisplatin, Clarithromycin, Clavulansäure, Clomipramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxycyclin, Enalapril, Ephedrin, Epinephrin, Ergocaiciferol, Ergotamin, Erythromycin, Östradiol, Ethinylöstradiol, Etoposid, Eucalyptus globulus, Famotidin, Felodipin, Fenofibrat, Fenoterol, Fentanyl, Flavinmononukleotid, Fluconazol, Flunarizin, Fluorouracil, Fluoxetin, Flurbiprofen, Furosemid, Gallopamil, Gemfibrozil, Ginkgo biloba, Glibenclamid, Glipizid, Clozapin, Glycyrrhiza glabra, Griseofulvin, Guaifenesin, Haloperidol, Heparin, Hyaluronsäure, Hydrochlorthiazid, Hydrocodon, Hydrocortison, Hydromorphon, Ipratropiumhydroxid, Ibuprofen, Imipenem, Indomethacin, lohexol, lopamidol, Isosorbiddinitrat, Isosorbidmononitrat, Isotretinoin, Ketotifen, Ketoconazol, Ketoprofen, Ketorolac, Labetalol, Lactulose, Lecithin, Levocarnitin, Levodopa, Levoglutamid, Levonorgestrel, Levothyroxin, Lidocain, Lipase, Imipramin, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, N-Methylephedrin, Naftidrofixryl, Naproxen, Neomycin, Nicardipin, Nicergolin, Nikotinamid, Nikotin, Nikotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Paroxetin, Penicilline, Phenobarbital, Pentoxifyllin, Phenoxymethylpenicillin, Phenylephrin, Phenylpropanolamin, Phenytoin, Physostigmin, Piroxicam, Polymyxin B, Povidoniodin, Pravastatin, Prazepam, Prazosin, Prednisolon, Prednison, Bromcriptin, Propafenon, Propranolol, Proxyphyllin, Pseudoephedrin, Pyridoxin, Chinidin, Ramipril, Ranitidin, Reserpin, Retinol, Riboflavin, Rifampicin, Rutosid, Saccharin, Salbutamol, Salcatonin, Salicylsäure, Simvastatin, Somatotropin, Sotalol, Spironolacton, Sucralfat, Sulbactam, Sulfamethoxazol, Sulfasalazin, Sulpirid, Tamoxifen, Tegafur, Teprenon, Terazosin, Terbutalin, Terfenadin, Tetracain, Tetracyclin, Theophyllin, Thiamin, Ticlopidin, Timolol, Tranexaminsäure, Tretinoin, Triamcinolonacetonid, Triamteren, Triazolam, Trimethoprim, Troxerutin, Uracil, Valproinsäure, Verapamil, Folininsäure, Zidovudin, Zopiclon, deren Enantiomeren, deren organischen und anorganischen Salzen, deren Hydraten und deren Mischungen, Hydrochlorthiazid, Flufenaminsäure, Mefenaminsäure, Bendroflumethiazid, Benzthiazid, Ethacrinsäure und Diaminopyrimidinen.

8. Verwendung nach Anspruch 4 bis 7, wobei der biologisch wirksame Stoff ein Therapeutikum ist und wobei das

System weiter einen oder mehrere pharmazeutisch annehmbare Hilfsstoffe umfasst und wobei die Beladung mit den Arzneimitteln zwischen 5 Gew.-% und 95 Gew.-% liegt.

## Revendications

1. Utilisation d'un hydrogel de dextrane expansible et dégradable *in vivo,* dans laquelle ledit hydrogel de dextrane est un dextrane modifié au moyen d'au moins un acrylate ou un méthacrylate (d'alkyle en $C_{1-8}$) et dans laquelle la dégradation *in vivo* se produit par clivage du squelette de dextrane et/ou par clivage de liaisons de réticulation à l'intérieur de l'hydrogel de dextrane, en tant que composant expansible d'un système de libération d'agent biologique par explosion contrôlée en fonction du temps ou d'un système de délivrance d'un agent biologique par impulsions comprenant au moins un agent biologiquement actif et une membrane externe de polymères ou de lipides, qui est perméable aux ions et à l'eau mais imperméable à l'agent biologique, dans laquelle la libération ou la délivrance de l'agent biologique commence après un temps de latence se situant entre 1 heure et 2 semaines.

2. Utilisation d'un hydrogel de dextrane expansible et dégradable *in vivo,* dans laquelle ledit hydrogel de dextrane est un dextrane modifié au moyen d'au moins un acrylate ou un méthacrylate (d'hydroxyalkyle en $C_{1-8}$), et dans laquelle la dégradation se produit par clivage de liaisons de réticulation à l'intérieur de l'hydrogel de dextrane, en tant que composant expansible d'un système de libération d'agent biologique par explosion contrôlée en fonction du temps ou d'un système de délivrance d'un agent biologique par impulsions comprenant au moins un agent biologiquement actif et une membrane externe de polymères ou de lipides, qui est perméable aux ions et à l'eau mais imperméable à l'agent biologique, dans laquelle la libération ou la délivrance de l'agent biologique commence après un temps de latence se situant entre 1 heure et 2 semaines.

3. Utilisation selon la revendication 1 ou la revendication 2, dans laquelle ledit agent biologique est choisi dans le groupe constitué par des médicaments thérapeutiques et prophylactiques et des molécules synthétiques, des protéines, des acides nucléiques, des vitamines, des hormones, des nutriments, des arômes, des agents antimicrobiens, des insecticides, des fongicides, des herbicides, des fertilisants et des pesticides.

4. Utilisation selon la revendication 3, dans laquelle ledit agent biologique est un agent thérapeutique choisi dans le groupe constitué par des médicaments anti-inflammatoires non stéroïdiens, des promoteurs de réparation osseuse, des hydrates de carbone, des agents antinéoplasiques, des agents antiangiogéniques, des agents vasoactifs, des anticoagulants, des immunomodulateurs, des agents cytotoxiques, des agents antiviraux et des neurotransmetteurs.

5. Utilisation selon la revendication 3, dans laquelle ledit agent biologique est une protéine thérapeutique active choisie dans le groupe constitué par des facteurs de croissance fibroblastiques, des facteurs de croissance épidermiques, des facteurs de croissance dérivés des plaquettes, des facteurs de croissance dérivés des macrophages, tels que les facteurs stimulant la croissance et le développement de colonies de granulocytes et de macrophages, des facteurs ciliaires neurotrophiques, l'activateur tissulaire du plasminogène, les facteurs stimulant les cellules B, le facteur d'induction du cartilage, des facteurs de différenciation, des facteurs libérant l'hormone de croissance, l'hormone de croissance humaine, des facteurs de croissance des hépatocytes, des immunoglobulines, des facteurs de croissance analogues à l'insuline, des interleukines, des cytokines, des interférons, des facteurs de nécrose des tumeurs, des facteurs de croissance des nerfs, des facteurs de croissance des cellules endothéliales, l'extrait du facteur ostéogénique, les facteurs de croissance des cellules T, des inhibiteurs de croissance des tumeurs et des fragments de ceux-ci.

6. Utilisation selon la revendication 3, dans laquelle ledit agent biologique est un agent antimicrobien choisi dans le groupe constitué par des phénols halogénés, des diphényléthers chlorés, des aldéhydes, des alcools, des acides carboxyliques et leurs dérivés, des composés organométalliques, des composés iodés, des monoamines et des polyamines, des composés de sulfonium et de phosphonium ; des composés mercapto ainsi que leurs sels de métaux alcalins, de métaux alcalino-terreux et de métaux lourds ; des urées ; des dérivés isothia- et benzisothiazolone.

7. Utilisation selon la revendication 3, dans laquelle ledit agent biologique est un agent thérapeutique choisi dans le groupe constitué par l'acébutolol, l'acétylcystéine, l'acide acétylsalicylique, l'acyclovir, l'alfuzosine, l'alprazolam, l'alfacalcidol, l'allantoïne, l'allopurinole, l'alvérine, l'ambroxol, l'amikacine, l'amiloride, l'acide aminoacétique, l'amiodarone, l'amitriptyline, l'amlodipine, l'amoxicilline, l'ampicilline, l'acide ascorbique, l'aspartame, l'astémizole, l'aténolol, la béclométhasone, le bensérazide, le benzalkonium hydrochlorure, la benzocaïne, l'acide benzoïque, la

bétaméthasone, le bézafibrate, la biotine, le bipéridène, le bisoprolol, le bromazépam, la bromhexine, la bromocriptine, le budésonide, le bufexamac, le buflomédil, la buspirone, la caféine, le camphre, le captopril, la carbamazépine, le carbidopa, le carboplatine, le céfachlore, la céfalexine, le céfatroxil, la céfazoline, le céfixime, le céfotaxime, le ceftazidime, la ceftriaxone, le céfuroxime, les céphalosporines, la cétirizine, le chloramphénicol, le chlordiazépoxyde, la chlorhexidine, la chlorphéniramine, la chlortalidone, la choline, la cyclosporine, la cilastatine, la cimétidine, la ciprofloxacine, le cisapride, le cisplatine, la clarithromycine, l'acide clavulanique, la clomipramine, le clonazépam, la clonidine, le clotrimazole, la codéine, la choléstyramine, l'acide cromoglycique, la cyanocobalamine, la cyprotérone, le désogestrel, la dexaméthasone, le, dexpanthénol, le dextrométhorphane, le dextropropoxiphène, le diazépam, le diclofénac, la digoxine, la dihydrocodéine, la dihydroergotamine, la dihydroergotoxine, le diltiazem, la diphénhydramine, le dipyridamole, la dipyrone, le disopyramide, la dompéridone, la dopamine, la doxycycline, l'énalapril, l'éphédrine, l'épinéphrine, l'ergocalciférol, l'ergotamine, l'érythromycine, l'oestradiol, l'éthinyloestradiol, l'étoposide, l'Eucalyptus globulus, la famotidine, la félodipine, le fénofibrate, le fénotérol, le fentanyle, la flavine mononucléotide, le fluconazole, la flunarizine, le fluoro-uracile, la fluoxétine, le flurbiprofène, le furosémide, le gallopamil, le gemfibrozil, le Ginkgo biloba, le glibenclamide, le glipizide, la clozapine, le Glycyrrhiza glabra, la griséofulvine, la guaifénésine, l'halopéridol, l'héparine, l'acide hyaluronique, l'hydrochlorothiazide, l'hydrocodone, l'hydrocortisone, l'hydromorphone, l'ipratropium , hydroxyde, l'ibuprofène, l'imipénème, l'indométhacine, l'iohexol, l'iopamidol, l'isosorbide dinitrate, l'isosorbide mononitrate, l'isotrétinoïne, le kétotifène, le kétoconazole, le kétoprofène, le kétorolac, le labétalol, le lactulose, la lécithine, la lévocamitine, le lévodopa, le lévoglutamide, le lévonorgestrel, la lévothyroxine, la lidocaïne, la lipase, l'imipramine, le lisinopril, le lopéramide, le lorazépam, la lovastatine, la médroxyprogestérone, le menthol, le méthotrexate, le méthyldopa, la méthylprednisolone, le métoclopramide, le métoprolol, le miconazole, le midazolam, la minocycline, le minoxidil, le misoprostol, la morphine, la N-méthyléphédrine, le naftidrofuryle, le naproxène, la néomycine, la nicardipine, la nicergoline, la nicotinamide, la nicotine, l'acide nicotinique, la nifédipine, la nimodipine, le nitrazépam, la nitrendipine, la nizatidine, la noréthistérone, la norfloxacine, le norgestrel, la nortriptyline, la nystatine, l'ofloxacine, l'oméprazole, l'ondansétrone, la pancréatine, le panthénol, l'acide pantothénique, le paracétamol, la paroxétine, les pénicillines, le phénobarbital, la pentoxifylline, la phénoxyméthylpénicilline, la phényléphrine, la phénylpropanolamine, la phénitoïne, la physostigmine, le piroxicam, la polymyxine B, la povidone iodée, la pravastatine, le prazépam, la prazosine, la prednisolone, la prednisone, la bromocriptine, la propafénone, le propranolol, la proxyphylline, la pseudoéphédrine, la pyridoxine, la quinidine, le ramipril, la ranitidine, la réserpine, le rétinol, la riboflavine, la rifampicine, le rutoside, la saccharine, le salbutamol, la salcatonine, l'acide salicylique, la simvastatine, la somatotropine, le sotalol, la spironolactone, le sucralfate, le sulbactam, le sulfaméthoxazole, la sulfasalazine, le sulpiride, le tamoxifène, le tégafur, la téprénone, la térazosine, la terbutaline, la terfénadine, la trétracaïne, la tétracycline, la théophylline, la thiamine, la ticlopidine, le timolol, l'acide tranexamique, la trétinoïne, la triamcinolone acétonide, le triamtérène, le triazolam, le triméthoprime, la troxérutine, l'uracile, l'acide valproïque, le vérapamil, l'acide follinique, la zidovudine, le zopiclone, des énantiomères de ceux-ci, des sels organiques et inorganiques de ceux-ci, des hydrates de ceux-ci et des mélanges de ceux-ci, l'hydrochlorothiazide, l'acide flufénamique, l'acide méfénamique, le bendrofluméthiazide, le benzthiazide, l'acide éthacrinique, et les diaminopyrimidines.

**8.** Utilisation selon les revendications 4 à 7, dans laquelle ledit agent biologique est un médicament thérapeutique et dans laquelle la charge en médicament est comprise entre 5 % et 95 % en poids, et dans laquelle ledit système comprend en outre un ou plusieurs excipients pharmaceutiquement acceptables.

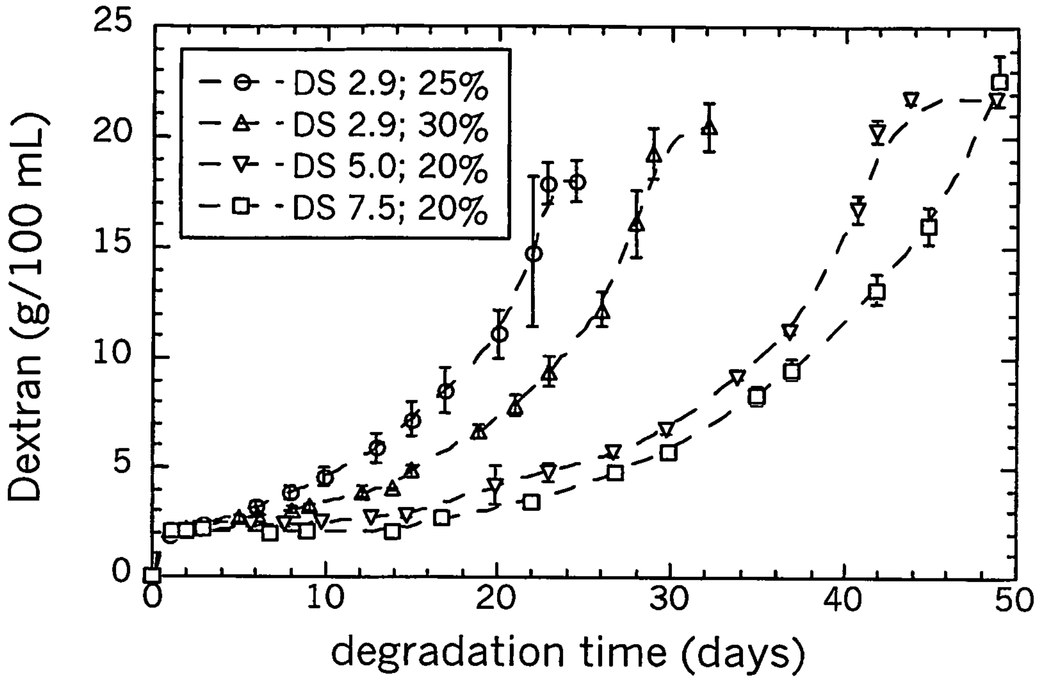

Dextran (g/100 mL)
degradation time (days)
DS 2.9; 25%
DS 2.9; 30%
DS 5.0; 20%
DS 7.5; 20%
0
5
10
15
20
25
0
10
20
30
40
50


Figure 1

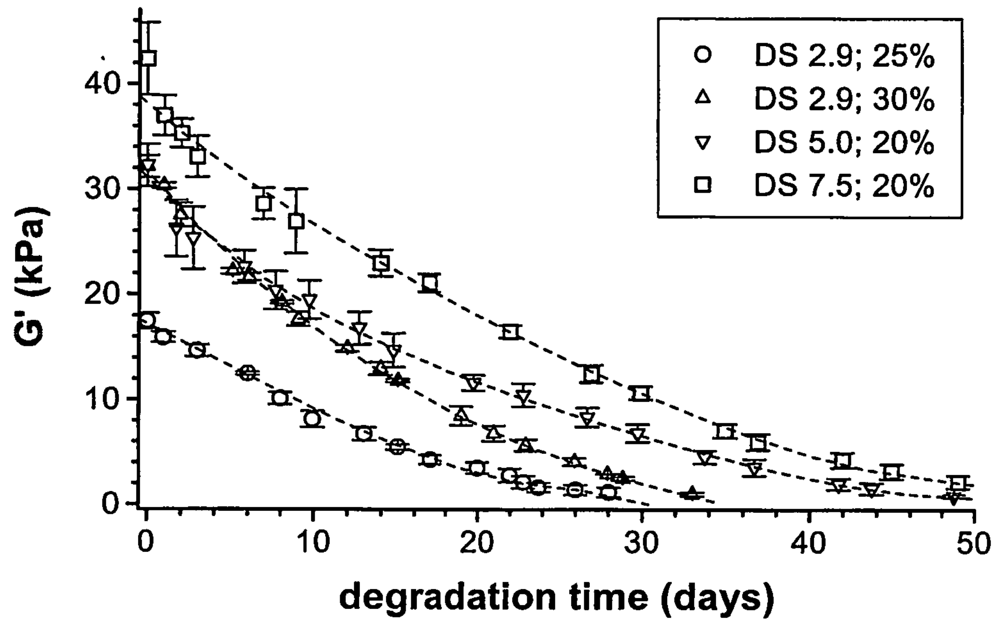
G' (kPa)
degradation time (days)
0
10
20
30
40
0
10
20
30
40
50
DS 2.9; 25%
DS 2.9; 30%
DS 5.0; 20%
DS 7.5; 20%

Figure 2

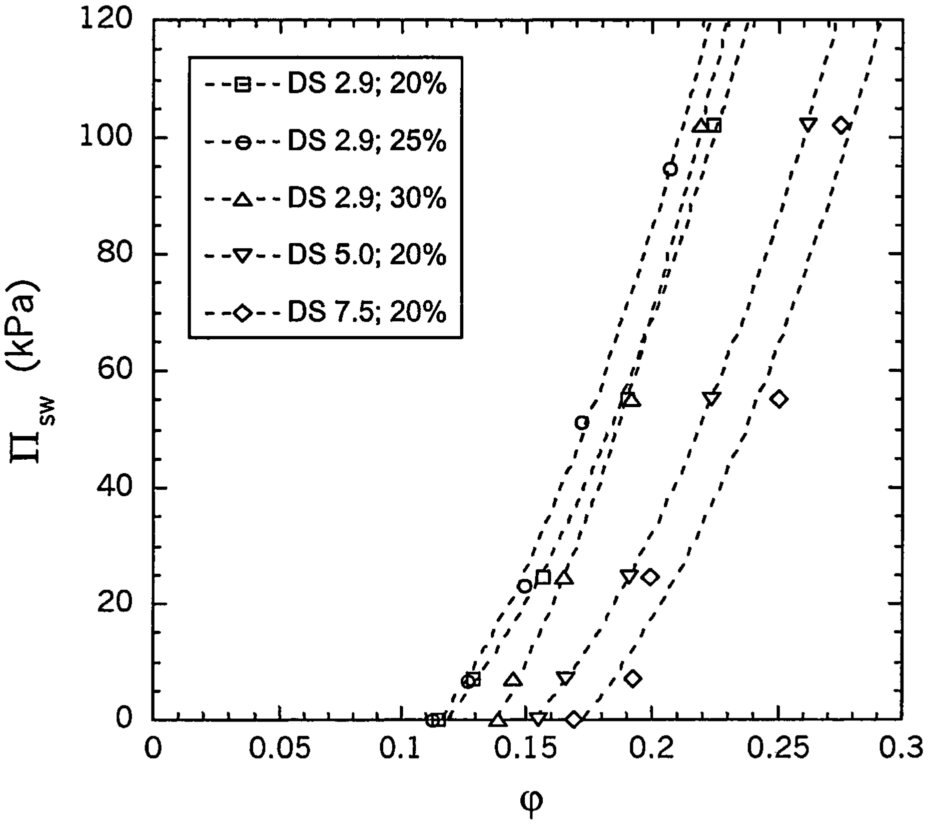
120
100
80
60
40
20
0
0
0.05
0.1
0.15
0.2
0.25
0.3
φ
$\Pi_{sw}$ (kPa)
DS 2.9; 20%
DS 2.9; 25%
DS 2.9; 30%
DS 5.0; 20%
DS 7.5; 20%

Figure 3

Figure 4

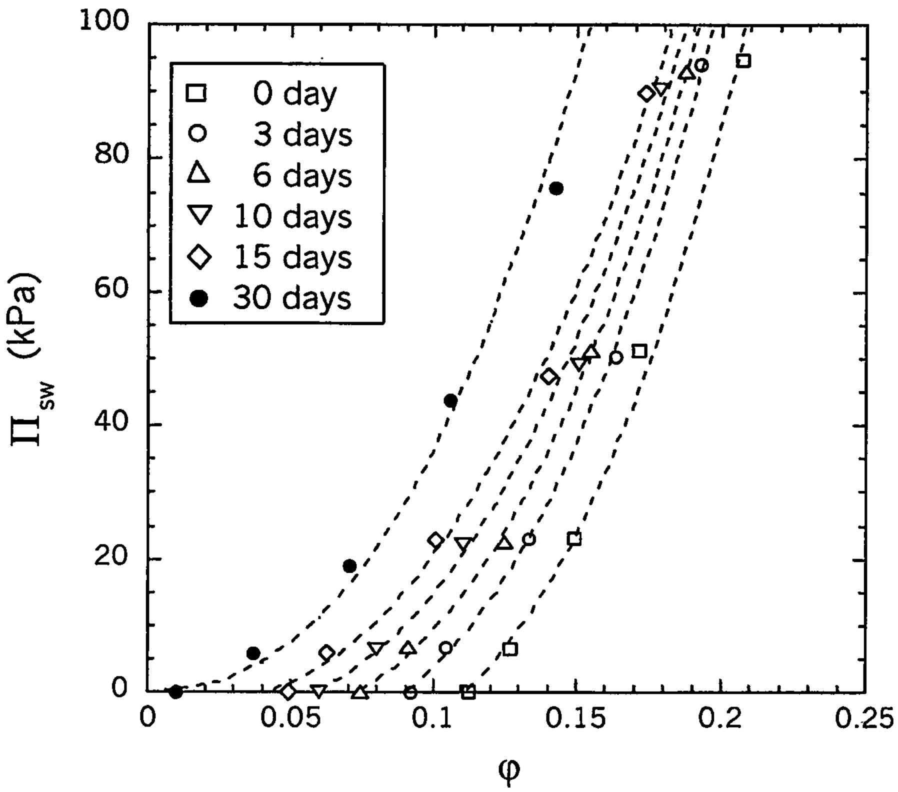
0 day
3 days
6 days
10 days
15 days
30 days
$\Pi_{sw}$ (kPa)
φ
0
20
40
60
80
100
0
0.05
0.1
0.15
0.2
0.25

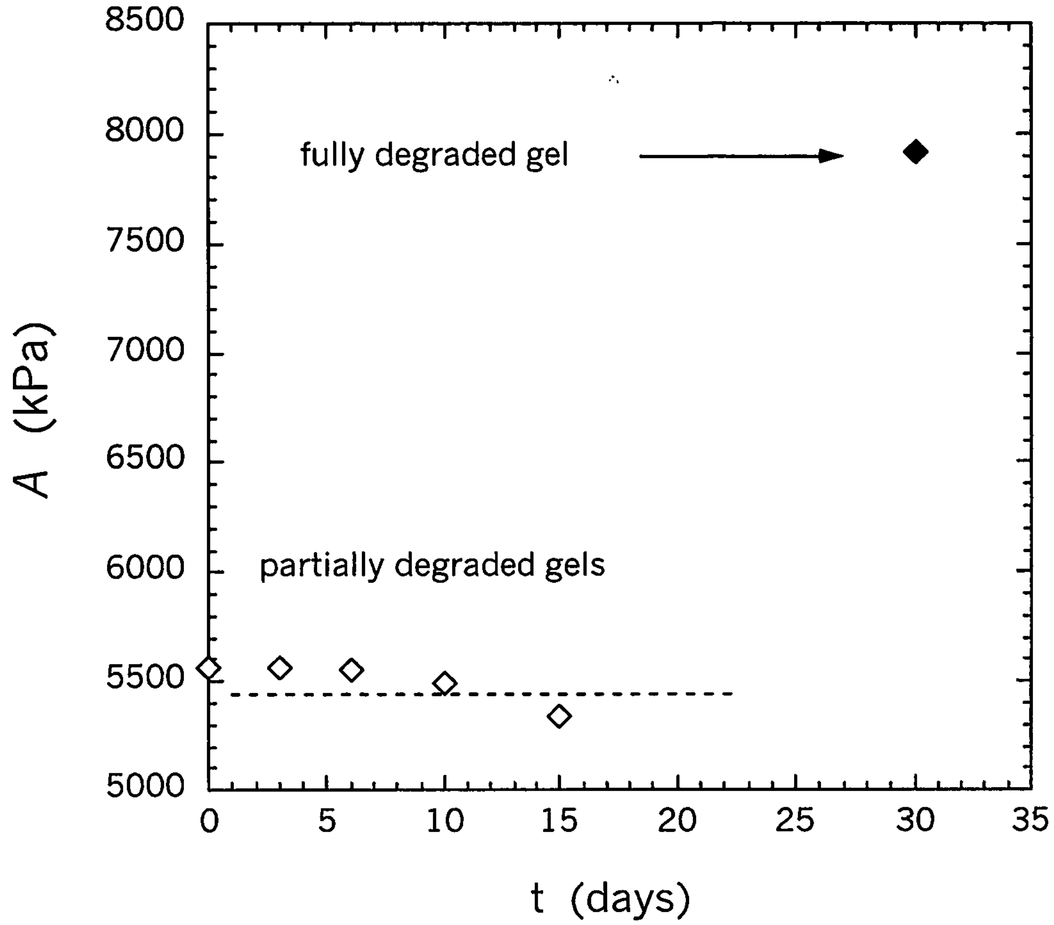
8500
8000
7500
7000
6500
6000
5500
5000
A (kPa)
fully degraded gel
partially degraded gels
0
5
10
15
20
25
30
35
t (days)

Figure 5

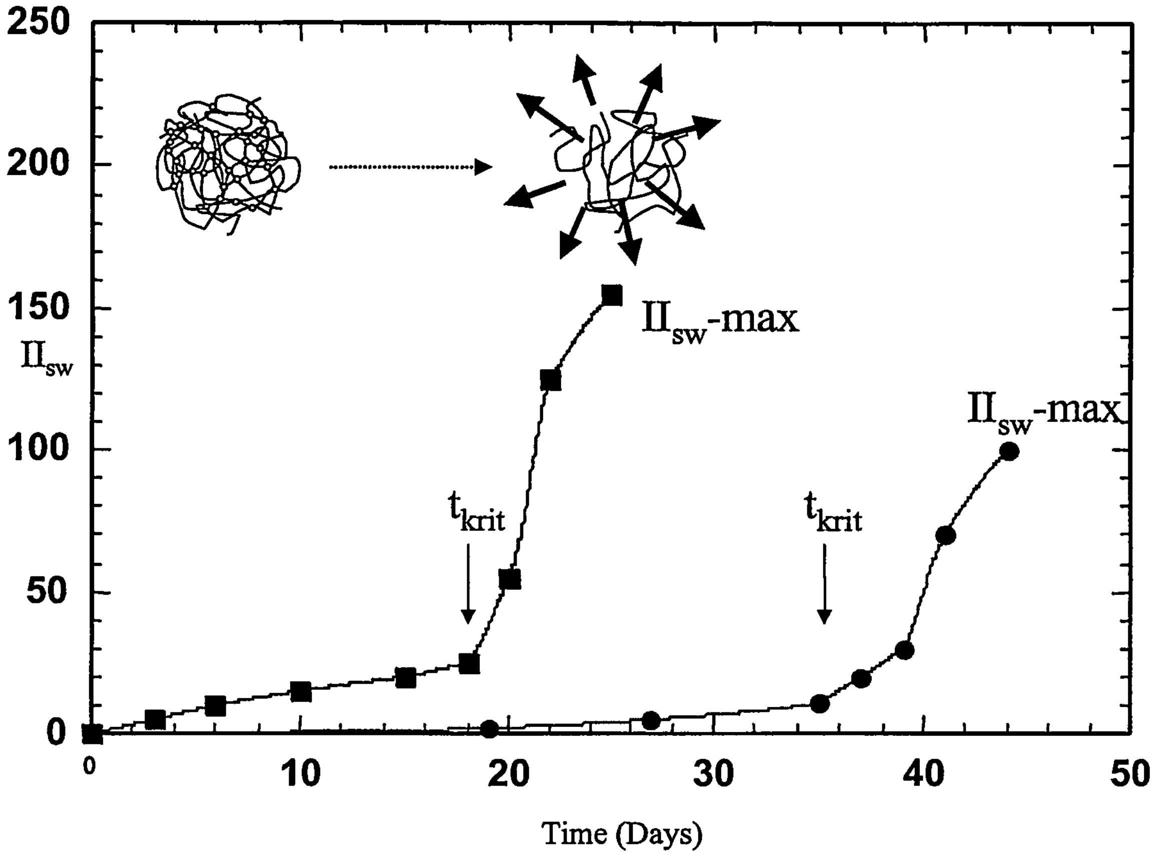
250
200
150
100
50
0
$\Pi_{sw}$
$\Pi_{sw}$-max
$\Pi_{sw}$-max
$t_{krit}$
$t_{krit}$
0
10
20
30
40
50
Time (Days)

Figure 6

Figure 7

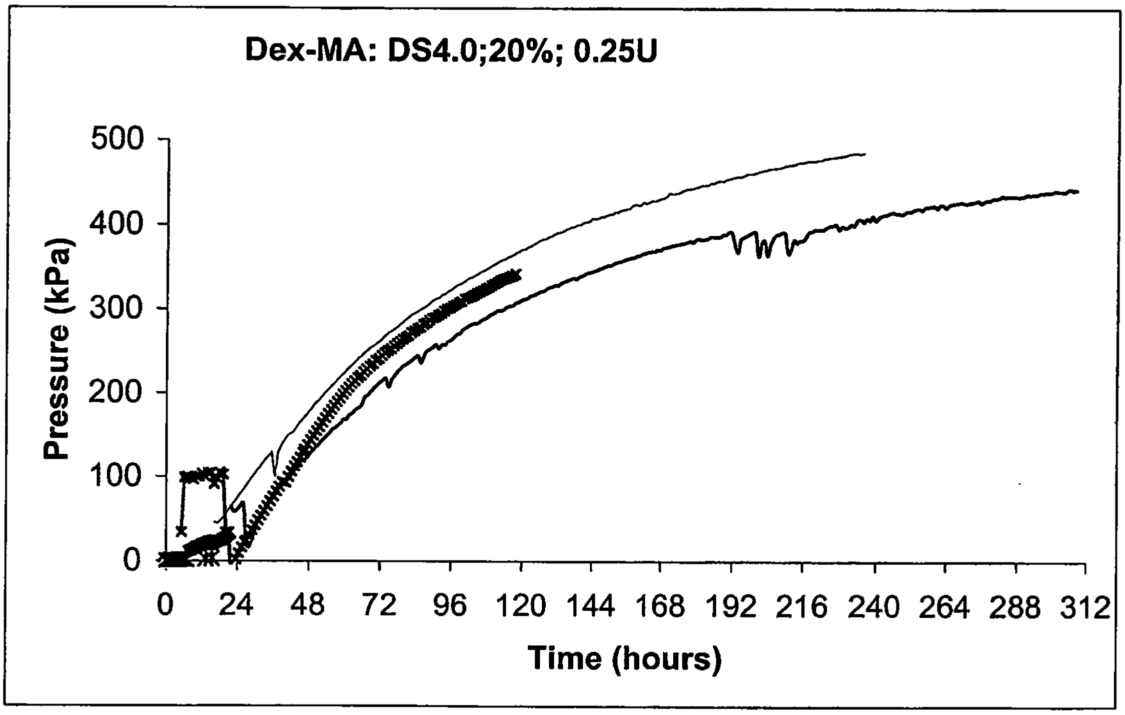
Dex-MA: DS4.0;20%; 0.25U
Pressure (kPa)
500
400
300
200
100
0
0 24 48 72 96 120 144 168 192 216 240 264 288 312
Time (hours)

Figure 8

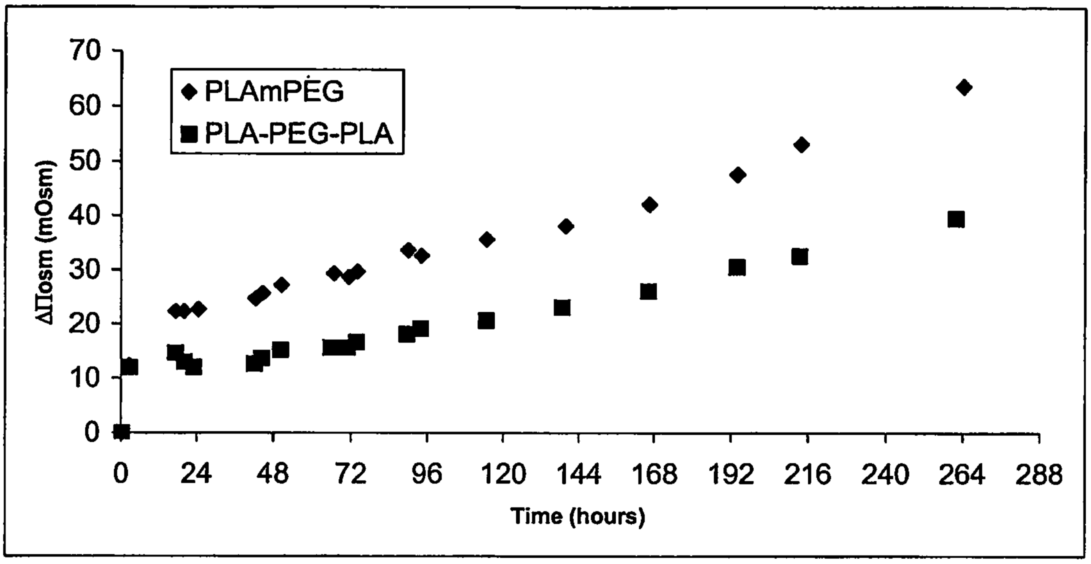


Figure 9

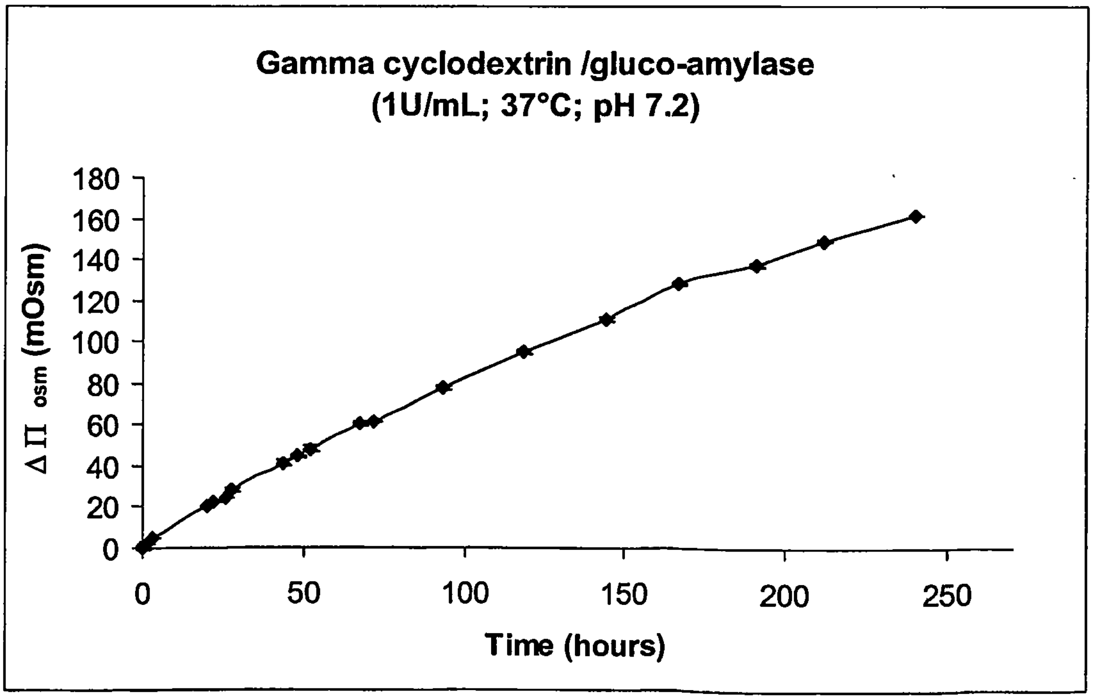

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4871549 A
- US 3247066 A
- US 3952741 A
- US 4933185 A
- US 5593697 A
- US 5654006 A

### Non-patent literature cited in the description


- **GENARRO A.L.** Remington: The Science and Practice of Pharmacy. vol. 47, 912-913
- **VAN DIJK et al.** *Macromolecules,* 1995, vol. 28, 6317-6322
- **G. AMIDON et al.** *Pharm. Res.,* 1995, vol. 12, 413-420
- **HORKAY et al.** *Macromolecules,* 1982, vol. 15, 1306-1310
- **NICHOL et al.** *Biochem J.,* 1967, vol. 102, 407-416
- **EDMOND et al.** *Biochem J.,* 1968, vol. 109, 569-576
- **DE SMEDT et al.** *Macromolecules,* 1995, vol. 28, 5082-5088
- **MEYVIS et al.** *J. Rheol.,* 1999, vol. 43, 933-950
- **P. G. DE GENNES.** Scaling concept in polymer physics. 1979